# FASCICULE DE BREVET EUROPEEN

(11) **EP 3 302 463 B1**
(45) Date de publication et mention de la délivrance du brevet: **25.03.2020**
(21) Numéro de dépôt: 16733153.7
(22) Date de dépôt: 27.05.2016
(51) Int. Cl.: A61K 31/352, C07D 311/60, A61P 27/02

(54) **UTILISATION DE 3-DÉSOXYANTHOCYANIDINES POUR LE TRAITEMENT DE MALADIES OCULAIRES**
VERWENDUNG VON 3-DEOXYANTHOCYANIDINEN ZUR BEHANDLUNG VON AUGENERKRANKUNGEN
USE OF 3-DEOXYANTHOCYANIDINS FOR TREATING OCULAR DISEASES

(30) Priorité: 27.05.2015 FR 1554761
(43) Date de publication de la demande: 11.04.2018
(73) Titulaire: Biophytis, 75001 Paris (FR); SORBONNE UNIVERSITE, 75006 Paris (FR); Centre National de la Recherche Scientifique (CNRS), 75016 Paris (FR); INSERM (Institut National de la Santé et de la Recherche Médicale), 75654 Paris Cedex 13 (FR)
(72) Inventeur: FONTAINE, Valérie, 94230 Cachan (FR); LAFONT, René, 75016 Paris (FR); SAHEL, José-Alain, 75013 Paris (FR); VEILLET, Stanislas, 91600 Savigny-sur-Orge (FR)
(74) Mandataire: Icosa
(86) Numéro de dépôt international: PCT/FR2016/051262
(87) Numéro de publication internationale: WO 2016/189260

(56) Documents cités:
- FR-A1- 2 975 008
- TANAKA ET AL.: "PURPLE RICE EXTRACT AND ANTHOCYANIDINS OF THE CONSTITUENTS PROTECT AGAINST LIGHT-INDUCED RETINAL DAMAGE IN VITRO AND IN VIVO", JOURNAL OF AGRICULTURAL FOOD CHEMISTRY, vol. 59, 2011, pages 528-536, XP002756236, cité dans la demande
- TANAKA ET AL.: "PURPLE RICE EXTRACT AND ITS CONSTITUENTS SUPPRESS ENDOPLASMIC RETICULUM STRESS-INDUCED RETINAL DAMAGE IN VITRO AND IN VIVO", LIFE SCIENCES, vol. 92, 2013, pages 17-25, XP002756237, cité dans la demande
- COSTANTINO L ET AL: "Authocyanidines as inhibitors of xanthine oxidase", PHARMAZIE,, vol. 50, no. 8, 1 janvier 1995 (1995-01-01), pages 573-574, XP009189396, ISSN: 0031-7144

## Description

### DOMAINE DE L'INVENTION

La présente invention concerne l'utilisation de composés de la famille des flavonoïdes, les anthocyanidines, en particulier des 3-désoxyanthocyanidines, pour le traitement, la prévention et/ou la stabilisation de maladies oculaires, en particulier pour le traitement, la prévention et/ou la stabilisation de la dégénérescence maculaire liée à l'âge (DMLA), la maladie de Stargardt, la rétinopathie pigmentaire et/ou la rétinopathie diabétique.

### ÉTAT DE LA TECHNIQUE

L'accumulation de lipofuscines dans les cellules rétiniennes, telles que les cellules de l'épithélium pigmentaire rétinien (EPR), est impliquée dans diverses maladies oculaires et apparait au cours du vieillissement. Les lipofuscines des cellules de l'EPR comprennent des lipides, des protéines et un mélange hétérogène de fluorophores, en particulier l'A2E (N-rétinyl-N-rétinylidène éthanolamine).

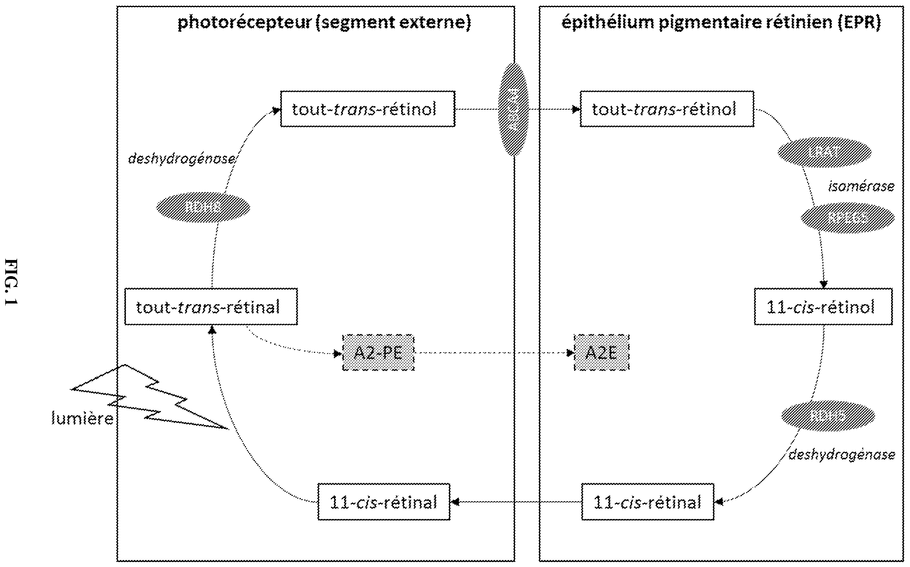

L'A2E est un sous-produit du cycle visuel (Figure 1), généré dans le segment externe des photorécepteurs sous forme d'un précurseur lié à un phospholipide. Lors du cycle visuel, le 11-*cis*-rétinal est isomérisé en tout-*trans*-rétinal sous l'effet de la lumière dans les photorécepteurs. Les cellules de l'EPR permettent de régénérer le 11-*cis*-rétinal à partir du tout-*trans*-rétinal. De plus, les cellules de l'EPR phagocytent et dégradent quotidiennement une partie des membranes des segments externes des photorécepteurs pour permettre le renouvellement de nouveaux disques à leur base. Toutefois, le tout-*trans*-rétinal n'est pas totalement régénéré en 11-*cis*-rétinal, une partie est transformée en A2E par la condensation de deux molécules de tout-*trans*-rétinal avec de l'éthanolamine. Avec l'âge, l'A2E s'accumule progressivement dans les cellules de l'EPR.

Il a été montré que l'accumulation de lipofuscines dans les cellules de l'EPR, en particulier l'accumulation d'A2E, provoque une augmentation de la mort des cellules de l'EPR. En effet, sous l'action de la lumière bleue et en présence d'oxygène, l'A2E génère des espèces réactives qui provoquent des dommages aux protéines, aux lipides et à l'ADN, et donc un stress oxydatif important dans les cellules vieillissantes de l'EPR (Sparrow JR et Cai B., Invest Ophthalmol Vis Sci, 2001, 42, 1356-1362 ; Sparrow JR et al., J Biol Chem, 2003, 278(20), 18207-18213). Les déchets ainsi formés s'accumulent et finissent par provoquer de place en place la mort des cellules de l'EPR, suivie par celle des photorécepteurs auxquels elles étaient associées.

Diverses maladies oculaires sont liées à l'accumulation de lipofuscines dans les cellules de l'EPR, telles que par exemple la DMLA ou la maladie de Stargardt.

La DMLA est une maladie dégénérative rétinienne chronique, évolutive et invalidante, qui touche le sujet âgé et dont l'origine est multifactorielle. C'est une cause de cécité irréversible chez les populations de personnes âgées, en particulier en Europe et en Amérique du Nord.

La DMLA affecte la partie centrale de la rétine, appelée macula, entraînant une grave déficience visuelle et la perte irréversible de la vision centrale. Les mécanismes physiopathologiques de la DMLA sont encore peu connus, mais il a été établi que l'accumulation progressive de lipofuscines, et la sénescence de l'EPR pouvaient être impliquées.

Le stade précoce de la DMLA est marqué par des dépôts, appelés Drüsen, qui n'affectent la vision que de façon marginale. Les phases ultérieures de la maladie comprennent deux formes sévères de DMLA : la forme sèche, aussi appelée atrophie géographique, et la forme humide, également connue sous le nom de forme exsudative ou forme néovasculaire. La forme sèche est plus fréquente que la forme humide, mais seule cette dernière bénéficie actuellement de traitements.

Des tentatives de prévention ou de traitement reposent actuellement sur des compléments alimentaires comprenant des composés antioxydants génériques, tels que par exemple le zinc, les vitamines A, C et E, avec une efficacité thérapeutique limitée.

Il existe donc un besoin pour de nouveaux composés actifs pour le traitement, la prévention et/ou la stabilisation de la DMLA, en particulier DMLA sèche, mais aussi pour le traitement, la prévention et/ou la stabilisation de maladies oculaires liées à l'accumulation de lipofuscines dans les cellules rétiniennes telles que la maladie de Stargardt.

À cet effet, l'utilisation d'anthocyanines issues d'extraits naturels a été rapportée (Liu et al., British J. Nutr., 2012, 108, 16-27 ; Wang et al., J. Sci. Food Agric., 2015, 95, 936-944).

Les anthocyanines appartiennent à la classe des composés polyphénoliques et font partie des pigments naturellement présents dans les fruits et les fleurs. La structure de ces composés comprend un cation flavylium lié à un glycoside (Schéma 1). Dans la nature, les anthocyanines sont basées sur 6 aglycones différents, liés à divers sucres, les plus communs étant le glucose, le galactose et l'arabinose. La partie aglycone des anthocyanines est appelée anthocyanidine.

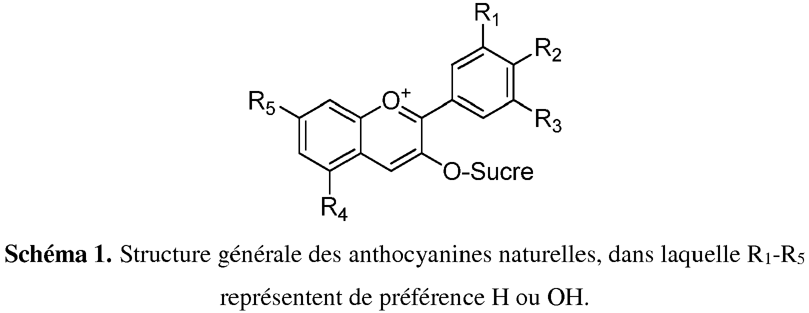

Sparrow et al. (WO2005/077176) ont également décrit l'utilisation d'anthocyanines pour le traitement ou la prévention de maladies oculaires liées à l'accumulation d'A2E dans les cellules de l'EPR. L'activité de certaines anthocyanines a été comparée avec leurs équivalent anthocyanidines. En particulier, la cyanidine, la pétunidine et la malvidine (Tableau 1) ont été évaluées et ont montré des activités de photoprotection équivalentes à celles obtenues pour les anthocyanines correspondantes, avec une survie cellulaire des cellules de l'EPR exposées à la lumière bleue allant de 30 à 70 % après incubation avec 100 µM de composés.

**Tableau 1 : Anthocyanidines naturelles testées pour leur activité photoprotectrice.**

| 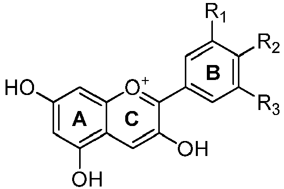 | **R₁** | **R₂** | **R₃** |
|---|---|---|---|
| cyanidine | OH | OH | H |
| delphinidine | OH | OH | OH |
| malvidine | OCH₃ | OH | OCH₃ |
| péonidine | OCH₃ | OH | H |
| pétunidine | OH | OH | OCH₃ |

D'autres études ont également montré que l'utilisation d'anthocyanidines naturelles pouvait avoir un effet photoprotecteur sur les cellules de l'EPR (Tanaka et al., J. Agric. Food Chem., 2011, 59, 528-536 ; Tanaka et al., Life Sciences, 2013, 92, 17-25 ; FR2996773). En particulier, la cyanidine, la delphinidine, la péonidine et la malvidine ont été évaluées dans ces études. La cyanidine et la delphinidine semblent avoir un effet photoprotecteur, contrairement à la malvidine. Des résultats contradictoires ont été obtenus concernant la péonidine.

Cependant, d'autres études ont au contraire conclu à l'inefficacité des anthocyanidines pour la survie cellulaire des cellules de l'EPR, en particulier pour la cyanidine, la delphinidine, la péonidine, la pétunidine et la malvidine (Hanneken et al., Invest. Ophthal. Visual Sci., 2006 ; 47(7), 3164-3177 ; Majumdaar et Srirangam, J. Pharm. Pharmacol., 2010, 62, 951-965).

L'art antérieur donne donc des résultats contradictoires concernant l'efficacité des anthocyanidines dans la photoprotection des cellules de l'EPR.

Malgré les préjugés négatifs existants, la Demanderesse a conduit des études approfondies sur l'activité d'anthocyanidines naturelles et non-naturelles pour la photoprotection des cellules de l'EPR.

D'une manière surprenante, la Demanderesse a mis en évidence que les 3-désoxyanthocyanidines comportant au moins un groupe hydroxyle sur le cycle A et sur le cycle B, présentent une très bonne activité photoprotectrice. En particulier, la présente invention concerne l'utilisation de 3-désoxyanthocyanidines de Formule I :

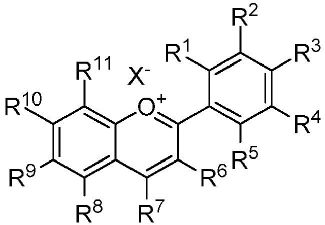

dans laquelle **R¹, R², R³, R⁴, R⁵, R⁶, R⁷, R⁸, R⁹, R¹⁰, R¹¹** et **X⁻** sont tels que définis ci-dessous, en particulier **R⁶** est différent d'un hydroxyle, au moins un de **R¹, R², R³, R⁴** ou **R⁵** représente un hydroxyle et au moins un de **R⁸, R⁹, R¹⁰** ou **R¹¹** représente un hydroxyle ; pour le traitement, la prévention et/ou la stabilisation de la DMLA, la maladie de Stargardt, la rétinopathie pigmentaire et/ou la rétinopathie diabétique.

La Demanderesse a montré que ces composés sont efficaces dans un modèle cellulaire de phototoxicité induite par l'association d'un traitement par l'A2E et d'une illumination par la lumière bleue sur des cultures primaires d'EPR. La Demanderesse a également montré que ces composés assurent une photoprotection dans un modèle *in vivo.*

### RÉSUMÉ

L'invention concerne donc un composé de Formule I 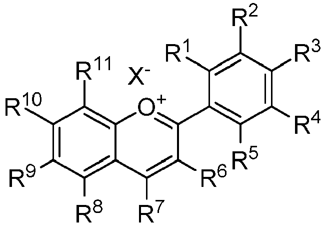 dans laquelle
**R¹, R², R³, R⁴** et **R⁵** représentent chacun indépendamment un groupement sélectionné parmi hydrogène, halo, hydroxyle, alkoxy, aryloxy, hétéroaryloxy, acyloxy, alkyle, aryle, aralkyle, alkylaryle, alcényle, nitro, nitrile, amino, avec la condition qu'au moins un de **R¹, R², R³, R⁴** ou **R⁵** représente un hydroxyle ;
**R⁶** représente un groupement sélectionné parmi hydrogène, halo, alkoxy, aryloxy, hétéroaryloxy, acyloxy, alkyle, aryle, aralkyle, alkylaryle, alcényle, nitro, nitrile, amino ;
**R⁷** représente un groupement sélectionné parmi hydrogène, halo, hydroxyle, alkoxy, aryloxy, hétéroaryloxy, acyloxy, alkyle, aryle, aralkyle, alkylaryle, alcényle, nitro, nitrile, amino ;
**R⁸, R⁹, R¹⁰** et **R¹¹** représentent chacun indépendamment un groupement sélectionné parmi hydrogène, halo, hydroxyle, alkoxy, aryloxy, hétéroaryloxy, acyloxy, alkyle, aryle, aralkyle, alkylaryle, alcényle, nitro, nitrile, amino, avec la condition qu'au moins un de **R⁸, R⁹, R¹⁰** ou **R¹¹** représente un hydroxyle ;
**X⁻** représente un anion sélectionné parmi : anion dérivé d'un acide minéral tel que par exemple un anion bromure, chlorure, borotétrafluorure ou perchlorure ; anion dérivé d'un acide organique, tel que par exemple un anion acétate, borate, citrate, tartrate, bisulfate, sulfate ou phosphate ; ou un anion dérivé d'un groupement sulfate ou sulfonate ;
pour son utilisation dans le traitement, la prévention et/ou la stabilisation de la DMLA, la maladie de Stargardt, la rétinopathie pigmentaire et/ou la rétinopathie diabétique.

Selon un mode de réalisation, dans le composé de formule I, **R⁶** représente un atome d'hydrogène.

Selon un mode de réalisation, le composé de formule I est de Formule la 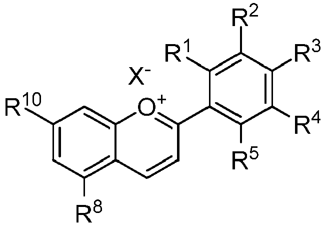 dans laquelle **R¹, R², R³, R⁴, R⁵, R⁸, R¹⁰** et **X⁻** sont tels que définis ci-dessus.

Selon un mode de réalisation, dans le composé de l'invention,
**R¹, R², R³, R⁴** et **R⁵** représentent chacun indépendamment un groupement sélectionné parmi hydrogène, hydroxyle et alkoxy, avec la condition qu'au moins un de **R¹, R², R³, R⁴** ou **R⁵** représente un hydroxyle ; et
**R⁸** et **R¹⁰** représentent chacun indépendamment un groupement sélectionné parmi hydrogène, hydroxyle et alkoxy, avec la condition qu'au moins un de **R⁸** ou **R¹⁰** représente un hydroxyle.

Selon un mode de réalisation, le composé de formule I est de Formule Ib 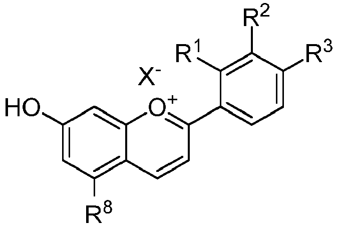 dans laquelle **R¹, R², R³, R⁸** et **X⁻** sont tels que définis ci-dessus.

Selon un mode de réalisation, dans le composé de l'invention, **R⁸** représente un atome d'hydrogène.

Selon un mode de réalisation, le composé pour son utilisation selon l'invention est sélectionné parmi :
chlorure de 2',7-dihydroxy-4'-méthoxy-flavylium ;
chlorure de 2',3',7-trihydroxy-4'-méthoxy-flavylium ;
chlorure de 3',7-dihydroxy-4'-méthoxy-flavylium ;
chlorure de 4',5,7-trihydroxy-flavylium ;
chlorure de 3',5,7-trihydroxy-4'-méthoxy-flavylium ;
chlorure de 3',4',5',5,7-pentadroxy-flavylium ;
chlorure de 3',4',5,7-tetrahydroxy-flavylium.

L'invention concerne également un composé sélectionné parmi :
chlorure de 2',7-dihydroxy-4'-méthoxy-flavylium ;
chlorure de 2',3',7-trihydroxy-4'-méthoxy-flavylium.

L'invention concerne également une composition pharmaceutique comprenant un composé sélectionné parmi chlorure de 2',7-dihydroxy-4'-méthoxy-flavylium et chlorure de 2',3',7-trihydroxy-4'-méthoxy-flavylium, en combinaison avec un véhicule pharmaceutiquement acceptable.

L'invention concerne également un médicament comprenant un composé sélectionné parmi chlorure de 2',7-dihydroxy-4'-méthoxy-flavylium et chlorure de 2',3',7-trihydroxy-4' -méthoxy-flavylium.

### DÉFINITIONS

Dans la présente invention, les termes ci-dessous sont définis de la manière suivante :
- « **acyloxy** » concerne un groupe -(C=O)-O-alkyle.
- « **alcényle** » concerne toute chaîne hydrocarbonée linéaire ou ramifiée, optionnellement substituée, portant au moins une double liaison, de 2 à 12 atomes de carbone, de préférence de 2 à 6 atomes de carbone ; tel que par exemple vinyle ou allyle.
- « **alkylaryle** » concerne un groupement alkyle-aryle-.
- « **alkyle** » concerne une chaîne hydrocarbonée linéaire ou ramifiée saturée, de 1 à 12 atomes de carbone, de préférence de 1 à 6 atomes de carbone, plus préférentiellement méthyle, éthyle, propyle, isopropyle, n-butyle, sec-butyle, isobutyle, tert-butyle.
- « **alkoxy** » concerne un groupe -O-alkyle.
- « **amino** » fait référence à un groupe -NH₂ ou à tout groupe dérivé de -NH₂ par substitution d'un ou plusieurs atomes d'hydrogène par un groupement organique aliphatique ou aromatique, substitué ou non substitué. De préférence, les groupes dérivés de -NH₂ sont des groupes alkylamino c'est-à-dire des groupes N-alkyle, comprenant les groupements monoalkylamino et dialkylamino.
- « **aralkyle** » concerne un groupement aryl-alkyle-.
- « **aryle** » concerne un système mono- ou polycyclique de 5 à 20, de préférence de 6 à 12, atomes de carbone possédant un ou plusieurs noyaux aromatiques, parmi lesquels on peut citer le groupe phényle, le groupe biphényle, le groupe 1-naphtyle, le groupe 2-naphtyle, le groupe tétrahydronaphtyle, le groupe indanyle, et le groupe binaphtyle. Le groupe aryle peut être substitué par 1 à 3 substituants choisis indépendamment les uns des autres, parmi un groupe hydroxyle, un groupe alkyle linéaire ou ramifié comportant 1, 2, 3 4, 5 ou 6 atomes de carbones, notamment le méthyle, l'éthyle, le propyle, le butyle, un groupe alcoxy ou un atome d'halogène, notamment brome, chlore et iode.
- « **aryloxy** » concerne un groupe -O-aryle.
- « **halo** » fait référence à fluoro, chloro, bromo, ou iodo.
- « **hétéroaryl** » concerne un groupe mono- ou polycyclique de 5 à 20, de préférence de 5 à 12, atomes de carbone possédant un ou plusieurs noyaux aromatiques, dans lequel un ou plusieurs atomes de carbone sont remplacés par un hétéroatome, de préférence N, O ou S, les hétéroatomes azote et soufre pouvant facultativement être oxydés et les hétéroatomes d'azote pouvant éventuellement être quaternisés. De tels cycles peuvent être condensés à un groupe aryle, cycloalkyle, hétéroaryle ou hétérocyclyle. Des exemples non limitatifs d'un tel groupe hétéroaryle sont les suivants: pyrrolyle, furanyle, thiophényle, pyrazolyle, imidazolyle, oxazolyle, isoxazolyle, thiazolyle, isothiazolyle, triazolyle, oxadiazolyle, thiadiazolyle, tétrazolyle, oxatriazolyle, thiatriazolyle, pyridinyle, pyrimidyle, pyrazinyle, pyridazinyle, oxazinyle, dioxinyle, thiazinyle, triazinyle, imidazo [2,1-b] [1, 3] thiazolyle, thiéno [3,2-b] furanyle, thiéno [3,2-b] thiophényle, thiéno [2,3-d] [1, 3] thiazolyle, thiéno [2,3-d] imidazolyle, tétrazolo [1,5-a] pyridinyle, indolyle, indolizinyle, iso-indolyle, benzofuranyle, isobenzofuranyle, benzothiophényle, isobenzothiophenyl, indazolyle, benzimidazolyle, 1,3-benzoxazolyle, 1,2-benzisoxazolyle, 2,1-benzisoxazolyle, 1,3-benzothiazolyle, 1, 2-benzoisothiazolyle, 2,1-benzoisothiazolyle, benzotriazolyle, 1,2,3-benzoxadiazolyle, 2,1, 3-benzoxadiazolyle, 1, 2,3-benzothiadiazolyle, 2,1,3-benzothiadiazolyle, thiénopyridinyle, purinyle, imidazo [1,2-a] pyridinyle, un 6-oxo-pyridazine-1 (6H) - yl, le 2-oxo- pyridine-1 (2H) - yle, 6-oxo-pyrudazin-1 (6H) -yl, le 2-oxo- pyridine-1 (2H) -yl, 1,3- benzodioxolyle, quinolinyle, isoquinolinyle, cinnolinyle, quinazolinyle, quinoxalinyle.
- « **hétéroaryloxy** » concerne un groupe -O-hétéroaryl.
- « **sujet** » fait référence à un animal, y compris un être humain. Au sens de la présente invention, un sujet peut être un « **patient** », à savoir une personne recevant des soins médicaux, subissant ou ayant subi un traitement médical, ou surveillée pour le développement d'une maladie.
- « **traitement** » signifie prévenir, réduire ou atténuer au moins un effet indésirable ou symptôme d'une maladie.
- « **prévention d'une maladie** » signifie éviter la survenue d'au moins un effet indésirable ou symptôme d'une maladie. Dans la présente invention, le terme «prévention» peut se référer à une prévention secondaire, à savoir la prévention de la réapparition d'un symptôme ou d'une rechute de la maladie.
- « **stabiliser une maladie** » signifie l'arrêt ou le ralentissement de l'aggravation d'au moins un effet indésirable ou symptôme d'une maladie. Il peut également se référer à l'action de réduire les conséquences d'une maladie une fois établie.
- « **quantité efficace** » fait référence à la quantité d'agent actif nécessaire et suffisante pour ralentir ou arrêter la progression, l'aggravation ou la détérioration d'un ou plusieurs symptômes d'une maladie ou de l'affection; ou le soulagement des symptômes d'une maladie ou d'un état; ou guérir la maladie ou affection.
- « **véhicule pharmaceutiquement acceptable** » fait référence à un excipient qui ne produit pas de réaction indésirable, allergique ou autre lorsqu'il est administré à un animal, de préférence un être humain. Il comprend tous les solvants, milieux de dispersion, revêtements, agents antibactériens et antifongiques, agents isotoniques, agents retardant l'absorption et composés similaires. Pour l'administration humaine, les préparations doivent répondre à des normes de stérilité, de sécurité générale et de pureté telles que requises par les offices règlementaire, tels que, par exemple, la FDA ou l'EMA.
- « **administration** » signifie fournir l'agent actif, seul ou en tant que partie d'une composition pharmaceutiquement acceptable, au sujet chez qui un symptôme ou la maladie doit être traité ou prévenu.

### DESCRIPTION DÉTAILLÉE

### Composés

La présente invention concerne des 3-désoxyanthocyanidines de Formule I 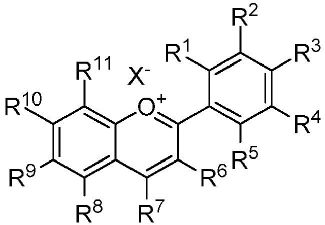 dans laquelle
**R¹, R², R³, R⁴** et **R⁵** représentent chacun indépendamment un groupement sélectionné parmi hydrogène, halo, hydroxyle, alkoxy, aryloxy, hétéroaryloxy, acyloxy, alkyle, aryle, aralkyle, alkylaryle, alcényle, nitro, nitrile, amino, avec la condition qu'au moins un de **R¹, R², R³, R⁴** ou **R⁵** représente un hydroxyle ;
**R⁶** représente un groupement sélectionné parmi hydrogène, halo, alkoxy, aryloxy, hétéroaryloxy, acyloxy, alkyle, aryle, aralkyle, alkylaryle, alcényle, nitro, nitrile, amino ;
**R⁷** représente un groupement sélectionné parmi hydrogène, halo, hydroxyle, alkoxy, aryloxy, hétéroaryloxy, acyloxy, alkyle, aryle, aralkyle, alkylaryle, alcényle, nitro, nitrile, amino ;
**R⁸, R⁹, R¹⁰** et **R¹¹** représentent chacun indépendamment un groupement sélectionné parmi hydrogène, halo, hydroxyle, alkoxy, aryloxy, hétéroaryloxy, acyloxy, alkyle, aryle, aralkyle, alkylaryle, alcényle, nitro, nitrile, amino, avec la condition qu'au moins un de **R⁸, R⁹, R¹⁰** ou **R¹¹** représente un hydroxyle ;
**X⁻** représente un anion sélectionné parmi : anion dérivé d'un acide minéral tel que par exemple un anion bromure, chlorure, borotétrafluorure ou perchlorure ; anion dérivé d'un acide organique, tel que par exemple un anion acétate, borate, citrate, tartrate, bisulfate, sulfate ou phosphate ; ou un anion dérivé d'un groupement sulfate ou sulfonate.

Dans un mode de réalisation particulier, **R¹, R², R³, R⁴** et **R⁵** représentent chacun indépendamment un groupement sélectionné parmi hydrogène, hydroxyle, alkoxy, aryloxy, hétéroaryloxy, acyloxy, avec la condition qu'au moins un de **R¹, R², R³, R⁴** ou **R⁵** représente un hydroxyle, de préférence avec la condition qu'au moins un de **R¹, R²** ou **R³** représente un hydroxyle.

Dans un mode de réalisation particulier, **R¹, R², R³, R⁴** et **R⁵** représentent chacun indépendamment un groupement sélectionné parmi hydrogène, hydroxyle, alkoxy, avec la condition qu'au moins un de **R¹, R², R³, R⁴** ou **R⁵** représente un hydroxyle, de préférence avec la condition qu'au moins un de **R¹, R²** ou **R³** représente un hydroxyle. De préférence, le groupement alkoxy est un groupement méthoxy.

Dans un mode de réalisation particulier, **R¹, R², R³, R⁴** et **R⁵** représentent chacun indépendamment un groupement sélectionné parmi hydrogène, hydroxyle, méthoxy, avec la condition qu'au moins un de **R¹, R², R³, R⁴** ou **R⁵** représente un hydroxyle, de préférence avec la condition qu'au moins un de **R¹, R²** ou **R³** représente un hydroxyle.

Dans un mode de réalisation particulier, **R¹** est un hydrogène ou un hydroxyle, de préférence **R¹** est un hydrogène.

Dans un mode de réalisation particulier, **R¹** est un hydrogène ou un hydroxyle.

Dans un mode de réalisation particulier, **R³** est un hydroxyle ou un alkoxy. Dans un mode de réalisation particulier, **R³** est un hydroxyle. Dans un mode de réalisation particulier, **R³** est un alkoxy, de préférence méthoxy.

Dans un mode de réalisation particulier, **R⁴** est un hydrogène. Dans un mode de réalisation particulier, **R⁵** est un hydrogène. Dans un mode de réalisation particulier, **R⁴** et **R⁵** sont des hydrogènes.

Dans un mode de réalisation particulier, **R⁶** représente un hydrogène, alkyle, alkoxy ou aryle. Dans un mode de réalisation particulier, **R⁶** représente un hydrogène.

Dans un mode de réalisation particulier, **R⁷** représente un hydrogène, un hydroxyl ou un alkoxy, de préférence **R⁷** représente un hydrogène.

Dans un mode de réalisation particulier, **R⁸, R⁹, R¹⁰** et **R¹¹** représentent chacun indépendamment un groupement sélectionné parmi hydrogène, hydroxyle, alkoxy, aryloxy, hétéroaryloxy, acyloxy, avec la condition qu'au moins un de **R⁸, R⁹, R¹⁰** ou **R¹¹** représente un hydroxyle, de préférence avec la condition qu'au moins **R⁸** ou **R¹⁰** représente un hydroxyle.

Dans un mode de réalisation particulier, **R⁸, R⁹, R¹⁰** et **R¹¹** représentent chacun indépendamment un groupement sélectionné parmi hydrogène, hydroxyle, alkoxy, avec la condition qu'au moins un de **R⁸, R⁹, R¹⁰** ou **R¹¹** représente un hydroxyle, de préférence avec la condition qu'au moins **R⁸** ou **R¹⁰** représente un hydroxyle. De préférence, le groupement alkoxy est un groupement méthoxy.

Dans un mode de réalisation particulier, **R⁸, R⁹, R¹⁰** et **R¹¹** représentent chacun indépendamment un groupement sélectionné parmi hydrogène, hydroxyle, avec la condition qu'au moins un de **R⁸, R⁹, R¹⁰** ou **R¹¹** représente un hydroxyle, de préférence avec la condition qu'au moins **R⁸** ou **R¹⁰** représente un hydroxyle.

Dans un mode de réalisation particulier, **R⁸** représente un hydrogène, un hydroxyle ou un alkoxy. Dans un mode de réalisation particulier, **R⁸** représente un hydrogène. Dans un mode de réalisation particulier, **R⁸** représente un hydroxyle. Dans un mode de réalisation particulier, **R⁶** et **R⁸** représentent des hydrogènes.

Dans un mode de réalisation particulier, **R⁹** représente un hydrogène, un hydroxyle ou un alkoxy. Dans un mode de réalisation particulier, **R⁹** représente un hydrogène.

Dans un mode de réalisation particulier, **R¹⁰** représente un hydrogène, un hydroxyle ou un alkoxy. Dans un mode de réalisation particulier, **R¹⁰** représente un hydroxyle.

Dans un mode de réalisation particulier, **R¹¹** représente un hydrogène, un hydroxyle ou un alkoxy. Dans un mode de réalisation particulier, **R¹¹** représente un hydrogène.

Dans un mode de réalisation particulier, **R⁹** et **R¹¹** représentent des hydrogènes. Dans un mode de réalisation particulier, **R⁸, R⁹** et **R¹¹** représentent des hydrogènes.

Dans un mode de réalisation particulier, **R⁸** et **R¹⁰** représentent des hydroxyles.

Dans un mode de réalisation particulier, **R⁸, R⁹** et **R¹¹** représentent des hydrogènes et **R¹⁰** représente un hydroxyle. Selon un mode de réalisation particulier, **R⁹** et **R¹¹** représentent des hydrogènes et **R⁸** et **R¹⁰** représentent des hydroxyles.

Dans un mode de réalisation particulier, **X⁻** représente un anion dérivé d'un acide minéral tel que par exemple un anion bromure, chlorure, borotétrafluorure ou perchlorure ; de préférence **X⁻** représente un chlorure ou un bromure ; plus préférentiellement **X⁻** représente un chlorure.

Dans un mode de réalisation particulier, **X⁻** représente anion dérivé d'un acide organique, tel que par exemple un anion acétate, borate, citrate, tartrate, bisulfate, sulfate ou phosphate.

Dans un mode de réalisation particulier, **X⁻** représente un anion dérivé d'un groupement sulfate ou sulfonate.

Selon un mode de réalisation, le composé de Formule I est de Formule I' 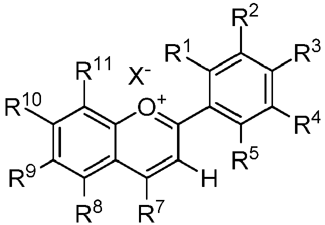 dans laquelle **R¹, R², R³, R⁴, R⁵, R⁷, R⁸, R⁹, R¹⁰, R¹¹** et **X⁻** sont tels que définis dans la formule I.

Selon un mode de réalisation, le composé de Formule I est de Formule la 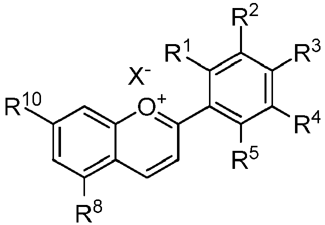 dans laquelle **R¹, R², R³, R⁴, R⁵, R⁸, R¹⁰** et **X⁻** sont tels que définis ci-dessus.

Selon un mode de réalisation particulier, dans la Formule la :
**R¹, R², R³, R⁴** et **R⁵** représentent chacun indépendamment un groupement sélectionné parmi hydrogène, hydroxyle et alkoxy, avec la condition qu'au moins un de **R¹, R², R³, R⁴** ou **R⁵** représente un hydroxyle ;
**R⁸** et **R¹⁰** représentent chacun indépendamment un groupement sélectionné parmi hydrogène, hydroxyle et alkoxy, avec la condition qu'au moins un de **R⁸** ou **R¹⁰** représente un hydroxyle.

Selon un mode de réalisation, le composé de Formule I est de Formule Ib 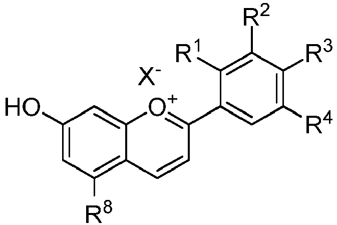 dans laquelle **R¹, R², R³, R⁴, R⁸** et **X⁻** sont tels que définis ci-dessus.

Selon un mode de réalisation particulier, dans la Formule Ib :
**R¹, R², R³** et **R⁴** représentent chacun indépendamment un groupement sélectionné parmi hydrogène, hydroxyle et alkoxy, avec la condition qu'au moins un de **R¹, R², R³** ou **R⁴** représente un hydroxyle ;
**R⁸** représente un groupement sélectionné parmi hydrogène, hydroxyle et alkoxy.

Selon un mode de réalisation particulier, dans la Formule Ib :
**R¹, R², R³** et **R⁴** représentent chacun indépendamment un groupement sélectionné parmi hydrogène, hydroxyle et méthoxy, avec la condition qu'au moins un de **R¹, R², R³** ou **R⁴** représente un hydroxyle ;
**R⁸** représente un hydrogène ou un hydroxyle.

Selon un mode de réalisation, les composés de Formule I sont ceux listés dans le tableau ci-dessous :

| **N° composé** | **Structure** | **Nom** |
|---|---|---|
| **1** | 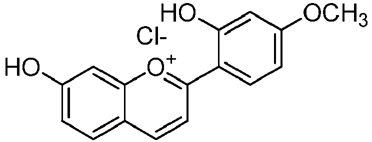 | chlorure de 2',7-dihydroxy-4'-méthoxy-flavylium |
| **2** | 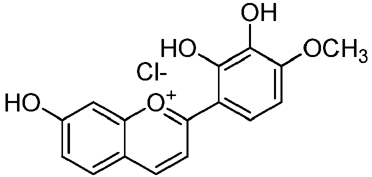 | chlorure de 2',3',7-trihydroxy-4'-méthoxy-flavylium |
| **3** | 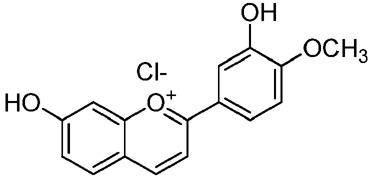 | chlorure de 3',7-dihydroxy-4'-méthoxy-flavylium |
| **4** | 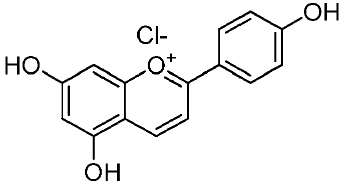 | chlorure de 4',5,7-trihydroxy-flavylium (apigéninidine) |
| **5** | 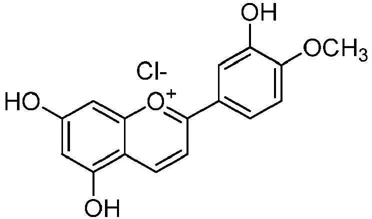 | chlorure de 3',5,7-trihydroxy-4'-méthoxy-flavylium (diosmétinidine) |
| **6** | 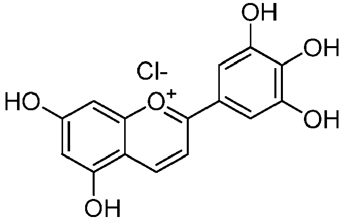 | chlorure de 3',4',5',5,7-pentadroxy-flavylium (tricétinidine) |
| **7** | 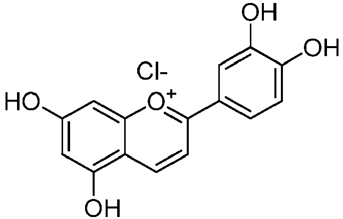 | chlorure de 3',4',5,7-tetrahydroxy-flavylium (lutéolinidine) |

Selon un mode de réalisation particulier, les composés de Formule I sont les composés n°1 et 2.

Selon un mode de réalisation, le composé de Formule I est un composé d'origine naturelle, tel que par exemple l'apigéninidine, la diosmétinidine, la tricétinidine ou la lutéolidine. Les composés d'origine naturelle peuvent être extraits de produits naturels, en particulier de plantes et/ou de fruits, ou bien être obtenus par synthèse chimique, que ce soit par synthèse totale ou par hémi-synthèse à partir de composés naturels, tels que par exemple les anthocyanines correspondantes.

Selon un autre mode de réalisation, le composé de Formule I est un composé non naturel. Les composés non-naturels peuvent être obtenus par synthèse chimique, soit par synthèse totale ou par hémi-synthèse.

Les composés de Formule I peuvent être préparés par des réactions connues de l'homme du métier.

### Utilisations

L'invention concerne une composition comprenant un composé de Formule I et un véhicule physiologiquement acceptable. Selon un mode de réalisation, la composition de l'invention est une composition pharmaceutique comprenant un composé de Formule I en combinaison avec un véhicule pharmaceutiquement acceptable.

L'invention concerne un médicament comprenant un composé de Formule I.

Les utilisations décrites ci-dessous concernent l'utilisation d'un composé de Formule I, d'une composition pharmaceutique ou d'un médicament selon la présente invention.

La présente invention concerne l'utilisation de composés de Formule I, pour le traitement, la prévention et/ou la stabilisation de maladies oculaires. Selon un mode de réalisation, l'invention concerne l'utilisation de composés de Formule I, pour le traitement, la prévention et/ou la stabilisation de maladies oculaires liée à l'accumulation de lipofuscines dans les cellules rétiniennes, en particulier pour le traitement, la prévention et/ou la stabilisation de la DMLA ou de la maladie de Stargardt.

Selon un mode de réalisation, l'invention concerne l'utilisation de composés de Formule I, pour le traitement, la prévention et/ou la stabilisation de la DMLA, la maladie de Stargardt, la rétinopathie pigmentaire et/ou la rétinopathie diabétique.

Selon un mode de réalisation, l'invention concerne l'utilisation des composés de Formule I pour le traitement, la prévention et/ou la stabilisation de la DMLA. Selon un mode de réalisation particulier, l'invention concerne l'utilisation des composés de Formule I pour le traitement, la prévention et/ou la stabilisation de formes intermédiaires de DMLA. Par « forme intermédiaire », il est fait référence au stade précoce de la DMLA, marqué par des dépôts appelés Drüsen, qui n'affectent la vision que de façon marginale. Selon un mode de réalisation particulier, l'invention concerne l'utilisation des composés de Formule I pour le traitement, la prévention et/ou la stabilisation de formes sévères de DMLA, en particulier les formes sèche et/ou humide de DMLA. Selon un mode de réalisation particulier, l'invention concerne l'utilisation des composés de Formule I pour le traitement, la prévention et/ou la stabilisation de la DMLA sèche. Selon un mode de réalisation particulier, l'invention concerne l'utilisation des composés de Formule I pour le traitement, la prévention et/ou la stabilisation de la DMLA humide.

Selon un mode de réalisation, l'invention concerne l'utilisation des composés de Formule I pour le traitement, la prévention et/ou la stabilisation de la maladie de Stargardt.

Selon un mode de réalisation, l'invention concerne l'utilisation des composés de Formule I pour le traitement, la prévention et/ou la stabilisation de la rétinopathie pigmentaire.

Selon un mode de réalisation, l'invention concerne l'utilisation des composés de Formule I pour le traitement, la prévention et/ou la stabilisation de la rétinopathie diabétique.

L'invention concerne également l'utilisation des composés de Formule I pour prévenir les dommages susceptibles d'être causés à la rétine par l'exposition aux rayonnements bleus. Par « rayonnement bleu », on entend les rayonnements correspondant à la bande bleue du spectre de la lumière visible, soit de longueur d'onde comprise entre 435 et 490 nm.

Selon un mode de réalisation, l'invention concerne l'utilisation des composés de Formule I pour réduire la mort cellulaire de l'épithélium pigmentaire rétinien (EPR).

Selon un mode de réalisation, l'invention concerne l'utilisation des composés de Formule I pour réduire ou prévenir l'accumulation de lipofuscine dans les cellules de l'EPR. Dans un mode de réalisation, la lipofuscine comprend A2E et/ou des formes isomères ou oxydées de l'A2E.

Selon un mode de réalisation, l'invention concerne l'utilisation des composés de Formule I pour stabiliser les membranes cellulaires de l'EPR. Dans un mode de réalisation, les membranes cellulaires peuvent être des membranes plasmatiques, lysosomales, nucléaires ou mitochondriales.

Selon un mode de réalisation, les utilisations mentionnées ci-dessus concernent un composé ou un mélange de composés de formule I.

Selon un mode de réalisation, les utilisations mentionnées ci-dessus concernent un ou des composés de Formule I en combinaison avec un autre agent actif. En particulier, l'agent actif peut être un antioxydant, tel que par exemple le zinc ou les vitamines A, C ou E.

Selon un mode de réalisation, le sujet est un animal, de préférence un mammifère, plus préférentiellement un humain.

Selon un mode de réalisation, le patient est diagnostiqué avec la DMLA, la maladie de de Stargardt, la rétinopathie pigmentaire et/ou la rétinopathie diabétique. Selon un autre mode de réalisation, le sujet est à risque de développer la DMLA, la maladie de de Stargardt, la rétinopathie pigmentaire et/ou la rétinopathie diabétique. Selon un mode de réalisation, le sujet présente une prédisposition génétique de développer la DMLA, la maladie de de Stargardt, la rétinopathie pigmentaire et/ou la rétinopathie diabétique. Selon un mode de réalisation, les composés de Formule I de l'invention sont administrés par voie intraoculaire. Par « voie intraoculaire » il est fait référence à une administration du composé directement dans l'intérieur de l'œil. L'intérieur de l'œil comprend en particulier la chambre antérieure, la chambre postérieure, le vitrée, la choroïde, la macula, la rétine, les vaisseaux sanguins et les nerfs qui vascularisent ou innervent la région postérieure de l'œil. Selon un mode de réalisation, les composés de Formule I sont administrés par voie intraoculaire dans le segment postérieur de l'œil. Selon un mode de réalisation, les composés de Formule I sont administrés par voie intravitréenne. Selon un autre mode de réalisation, les composés de Formule I sont administrés dans le compartiment sous-rétinien de l'œil. Selon un autre mode de réalisation, les composés de Formule I sont administrés par voie sous-conjonctivale.

Selon un mode de réalisation, les composés de Formule I sont administrés par voie topique. Des gouttes ou des bains peuvent être utilisés. Des méthodes d'iontophorèse connues de l'homme du métier peuvent être également utilisées pour favoriser l'absorption topique des composés de l'invention dans l'œil.

Selon un mode de réalisation, les composés de Formule I ne sont pas administrés par voie orale.

Selon un mode de réalisation, les composés de Formule I sont formulés dans une forme adaptée à l'injection. Dans un mode de réalisation, les composés de Formule I sont formulés sous la forme d'une solution, telle que par exemple une solution aqueuse stérile, une dispersion, une émulsion, une suspension ; ou sous une forme solide adaptée pour la préparation d'une solution ou d'une suspension par addition d'un liquide.

Selon un mode de réalisation, les composés de Formule I ont formulés de telle sorte qu'un relargage prolongé et/ou contrôlé puisse survenir. En particulier, les composés de Formule I peuvent être formulés sous la forme d'un implant ou dans une matrice biorésorbable. La matrice biorésorbable peut comprendre un carbomère ou un polymère. Le polymère peut être une microsphère biodégradable. Les composés de Formule I peuvent alternativement être formulés sous forme de liposomes. Toute formulation oculaire permettant d'obtenir un relargage prolongé et/ou contrôlé de la substance active connue de l'homme du métier peut être utilisée.

Selon un mode de réalisation, les composés de Formule I sont administrés à une fréquence d'au plus une fois par mois, de préférence une fois tous les 2 mois, plus préférentiellement une fois tous les 4 mois, encore plus préférentiellement une fois tous les 6 mois.

Selon un mode de réalisation, les composés de Formule I sont administrés par voie intravitréenne sous une forme permettant de libérer quotidiennement une dose allant de 0,01 mg à 1 mg par œil, de préférence 0,1 mg à 0,5 mg par œil.

### BRÈVE DESCRIPTION DES FIGURES

La **Figure 1** est un schéma résumant les principales étapes du cycle visuel.
Les **Figures 2** **et** **3** sont des graphiques représentant les électrorétinogrammes des souris une semaine après l'induction de la phototoxicité. L'onde A (Figure 2) représente l'activité électrique des photorécepteurs, et l'onde B (Figure 3) représente celle des cellules de la rétine interne. Les données ont été analysées statistiquement par une analyse de la variance suivie par un test de Dunnett. *p<0,05, **p<0,01, ***p<0,001.
La **Figure 4** est un graphique représentant le nombre de couches de photorécepteurs en fonction de la distance au nerf optique après injection intravitréenne de diosmétinidine.

### EXEMPLES

La présente invention se comprendra mieux à la lecture des exemples suivants qui illustrent non-limitativement l'invention.

### Exemple 1 : Synthèse des composés de l'invention

Composé 1. Le 2,4-dihydroxybenzaldéhyde (69 mg) et la 2'-hydroxy-4'-méthoxy-acétophénone (83 mg - 1 équivalent) sont mis en solution dans 500 µL d'éthanol. On ajoute 40 µL de chlorure de thionyle directement dans le milieu réactionnel qui devient rapidement très coloré. Après une heure d'agitation, le milieu réactionnel est évaporé et le résidu est précipité dans de l'acétate d'éthyle, filtré et séché pour donner le composé final (77 mg - rendement 50 %).

Composé 2. Le 2,4-dihydroxybenzaldéhyde (276 mg) et la 2',3'-dihydroxy-4'-méthoxy-acétophénone (364 mg - 1 équivalent) sont mis en solution dans 2 mL d'éthanol et 2 mL d'acétate d'éthyle. On ajoute 500 µL de chlorure de thionyle directement dans le milieu réactionnel qui devient rapidement coloré. Après une heure d'agitation, le milieu réactionnel est évaporé et le résidu est précipité dans de l'acétate d'éthyle, filtré et séché. Le solide est mis en solution dans un minimum de méthanol et est précipité par l'addition d'un volume équivalent de tertio-butyl-méthyl-éther (TBME). Le solide est filtré et séché. La procédure est renouvelée jusqu'à ce que la pureté soit correcte. Le rendement final est de 60 %.

Les composés obtenus ont des puretés > 95% (HPLC) et leur identité a été confirmée par spectroscopie SM et RMN. Les spectres de masse à haute résolution ont été réalisés sur un spectromètre LTQ Orbitrap-XL (ThermoFisher Scientific), équipé d'une source NSI (nano-ESI). Les spectres de résonance magnétique nucléaire (RMN) du proton (¹H) ont été effectués dans le DMSO-d6 + 1% CF₃COOD sur un appareil Bruker Avance DPX300 (300,16 MHz).

HRMS (Orbitrap).
Composé 1. m/z 269,0809 (M)+, calc. 269.0808 pour C₁₆H₁₃O₄, Δ = 0,054 ppm.
Composé 2. m/z 285,0757 (M)+, calc. 285,0757 pour C₁₆H₁₃O₅, Δ = -0,105 ppm.

**Spectres ¹H-RMN (δ ppm)**

| 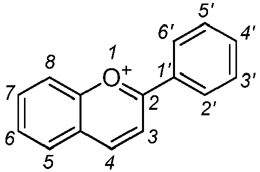 | 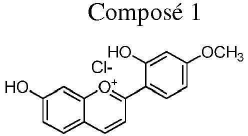 | 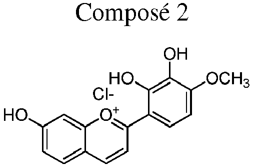 |
|---|---|---|
| 3-H | 7.44 (dd, 8.9, 2.2 Hz ) | 7.42 (dd, 8.9, 2.2 Hz) |
| 4-H | 9.11 (d, 9.0 Hz) | 9.09 (d, 9.0 Hz) |
| 5-H | 8.65 (d, 9.0 Hz) | 8.64 (d, 8.9 Hz) |
| 6-H | 6.77 (d, 2.3 Hz) | 6.94 (d, 9.4 Hz) |
| 8-H | 7.57 (d, 2.0 Hz) | 7.53 (d, 1.9 Hz) |
| 2'-H | 8.18 (d, 8.9 Hz) | 8.16 (d, 9.0 Hz) |
| 3'-H | 6.82 (dd, 9.2, 2.1 Hz) | 7.92 (d, 9.3 Hz) |
| 4'-OCH₃ | 3.92 (3H, s) | 4.01 (3H, s) |
| 5'-H | 8.36 (d, 9.1 Hz) | - |
| 6'-H | - | - |

### Exemple 2 : Test in vitro de l'activité photoprotectrice

### Méthode

Un modèle cellulaire de phototoxicité induite par l'association d'un traitement par l'A2E et d'une illumination par la lumière bleue sur des cultures primaires d'EPR, dans lequel a été mesurée la survie cellulaire, a été utilisé. Ce modèle utilise des cultures primaires d'épithélium pigmentaire rétinien de porc adulte.

Ce modèle permet notamment de réaliser le criblage de molécules visant à la découverte de nouveaux candidats pour un traitement de la forme sèche de la DMLA. Ce modèle est plus proche de la situation « physiologique » que les lignées cellulaires couramment utilisées dans la littérature, car les cellules utilisées contiennent des substances protectrices apportées par l'alimentation de l'animal et ne sont donc pas en situation de « carence », et leur perturbation est provoquée par l'apport d'A2E dans le milieu de culture.

Les cellules cultivées dans des plaques 96 puits ont été traitées pendant 48 heures avec les composés à tester (en solution 5 mM dans le DMSO) de façon à obtenir des concentrations finales de 5 ou 20 µM), dont les dernières 19 heures en présence d'A2E (concentration finale 30 µM). Les cellules pré-traitées sont ensuite illuminées pendant 50 min avec de la lumière bleue (470 nm) apportée par 96 LED W7113PBC/H (Kingbright) avec un angle d'émission de 16°, émettant 1440 mcd (millicandela) sous un courant de 8,6 mA. La survie des cellules est mesurée après 24 heures.

La survie et la mort cellulaires sont détectées 24 heures après l'induction de la phototoxicité par coloration des cellules au Hoechst (un marqueur nucléaire) et à l'éthidium (un marqueur des noyaux des cellules mortes). Des images de chaque puits sont acquises sur un microscope à fluorescence équipé d'une platine motorisée pilotée par le logiciel Métamorph, et la survie cellulaire est quantifiée par un programme de quantification dédié. Les expériences sont réalisées sur des microplaques de 96 puits en quadruplicate et chaque expérience est reproduite au minimum quatre fois.

Les résultats sont exprimés sous forme d'un ratio représentant le nombre de cellules vivantes dans les puits traités par les molécules à tester divisé par le nombre de cellules vivantes dans les puits contrôles (traités par le milieu de dilution sans A2E) et multiplié par 100. La valeur des contrôles traités par A2E mais sans molécule est de 39,7 ± 3,7.

### Résultats

Les composés de l'invention permettent d'obtenir des pourcentages de survie cellulaire très élevés, aussi bien à 20 µM qu'à 5 µM (Tableau 2).

**Tableau 2 : Survie cellulaire : composés de l'invention.**

| **Composés** | **20 µM** | **5 µM** |
|---|---|---|
| **1** | 89,9 ± 4,9 | ND |
| **2** | 86,7 ± 2,3 | ND |
| **3** | 77,0 ± 2,7 | ND |
| **4** | 89,5 ± 3,5 | 71 ± 3,6 |
| **5** | 90,8 ± 3,6 | 84,5 ± 1,4 |
| **6** | 99,3 ± 3,3 | 74,9 ± 4,2 |
| **7** | 93,6 ± 6,7 | 72 ± 4,4 |

| | | |
|---|---|---|
| *ND : non disponible* | | |

Pour comparaison, des 3-hydroxy-anthocyanidines ont été testées (Tableau 3).

**Tableau 3 : 3-hydroxy-anthocyanidines testées.**

| 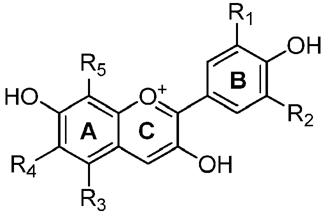 | **R₁** | **R₂** | **R₃** | **R₄** | **R₅** |
|---|---|---|---|---|---|
| cyanidine | OH | H | OH | H | H |
| delphinidine | OH | OH | OH | H | H |
| fisétinidine | H | OH | H | H | H |
| gossypétinidine | H | OH | OH | H | OH |
| guibourtinidine | H | H | H | H | H |
| malvidine | OCH₃ | OCH₃ | OH | H | H |
| péonidine | OCH₃ | H | OH | H | H |
| pétunidine | OH | OCH₃ | OH | H | H |
| quercétagétinidine | OH | H | OH | OH | H |

Les résultats de survie cellulaire en présence de 3-hydroxy-anthocyanidines sont reportés dans le tableau 4.

**Tableau 4 : Survie cellulaire : 3-hydroxy-anthocyanidines.**

| **Composés** | **20 µM** | **5 µM** |
|---|---|---|
| Cyanidine | 92,9 ± 1,9 | 56,4 ±8,8 |
| Delphinidine | 57,8 ± 5,6 | 40,5 ± 6,2 |
| Fisétinidine | 39,3 ± 3,2 | ND |
| Gossypétinidine | 36,5 ± 14,4 | ND |
| Guibourtinidine | 42,8 ± 6,9 | ND |
| Malvidine | 51,5 ± 6,9 | ND |
| Péonidine | 60,4 ± 8,5 | ND |
| Pétunidine | 67,2 ± 6,9 | ND |
| Quercétagétinidine | 54,3 ± 6,6 | ND |

Ces résultats montrent que la présence d'un groupement hydroxyle en position 3 des anthocyanidines diminue fortement l'efficacité de la photoprotection. Seule la cyanidine présente un effet photoprotecteur important à 20 µM. Toutefois, à plus faible concentration, son efficacité est réduite de manière importante, contrairement aux composés de l'invention.

Pour comparaison, des 3-désoxy-anthocyanidines ne portant pas au moins un hydroxyle libre sur le cycle A et sur le cycle B ont également été testées (Tableau 5).

**Tableau 5 : Survie cellulaire : 3-désoxy-anthocyanidines ne portant pas au moins un hydroxyle sur le cycle A ou sur le cycle B.**

| **Composés** | **20 µM** | **5 µM** |
|---|---|---|
| (a) | 39,2 ± 7,4 | ND |
| 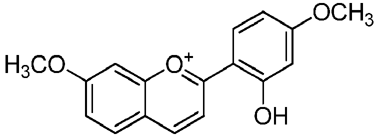 | | |
| (b) | 48,7 ± 5,5 | ND |
| 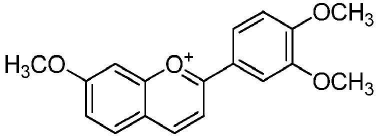 | | |
| (c) | 46,1 ± 4,7 | ND |
| 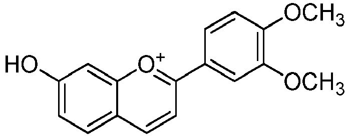 | | |

Les pourcentages de survie cellulaire obtenus à 20 µM sont inférieurs à 50 % ; la concentration 5 µM n'a donc pas été testée.

Les résultats obtenus montrent clairement que lorsqu'il n'y a pas au moins un groupe hydroxyle sur chacun des cycles A et B, l'efficacité de la photoprotection est bien moins importante qu'avec les composés de l'invention.

### Exemple 3 : Test in vivo de l'activité photoprotectrice chez la souris

Un modèle de souris génétiquement modifiée développé par Maeda et al. (Invest Ophthalmol. Vis. Sci., 2009, 50, 4917-4925) a été utilisé pour tester l'activité photoprotectrice des composés de l'invention.

Dans ce modèle de souris, deux gènes impliqués dans le cycle du pigment visuel (ABCA4 et Rdh8, voir Figure 1) sont inactivés, ce qui se traduit par une accumulation précoce d'A2E dans les yeux. Ce modèle animal est donc représentatif de la pathologie humaine.

Des souris âgées de 7 semaines ont été utilisées pour réaliser des injections intravitréennes unilatérales de diosmétinidine solubilisée (50 µM) dans du DMSO et diluée dans du PBS (1,2:100), afin d'obtenir une concentration dans le vitré de 100-130 µM. Du DMSO dilué dans le PBS a été injecté aux animaux témoins. Après 24 h à l'obscurité, les souris ont été soumises à une exposition à la lumière bleue (4000 lux, 1 h).

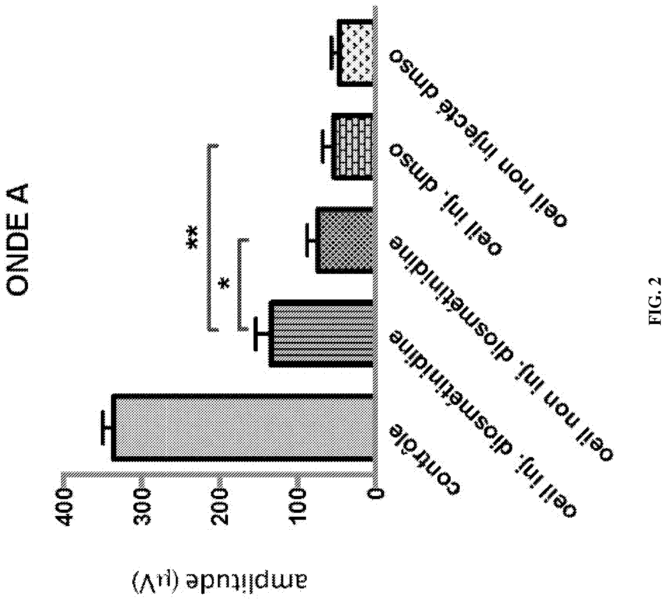

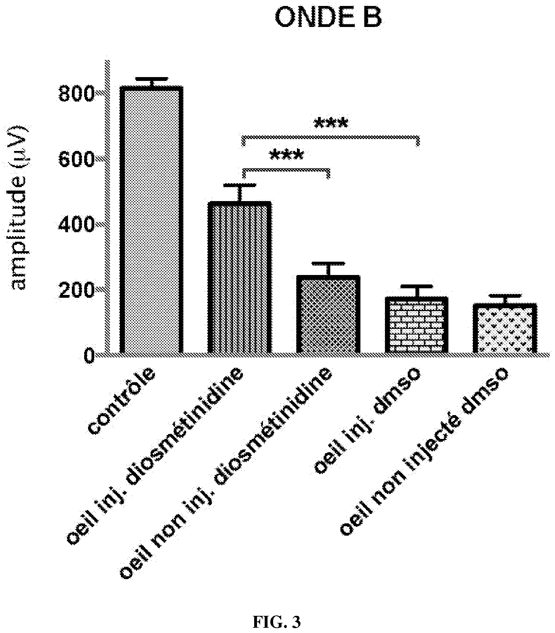

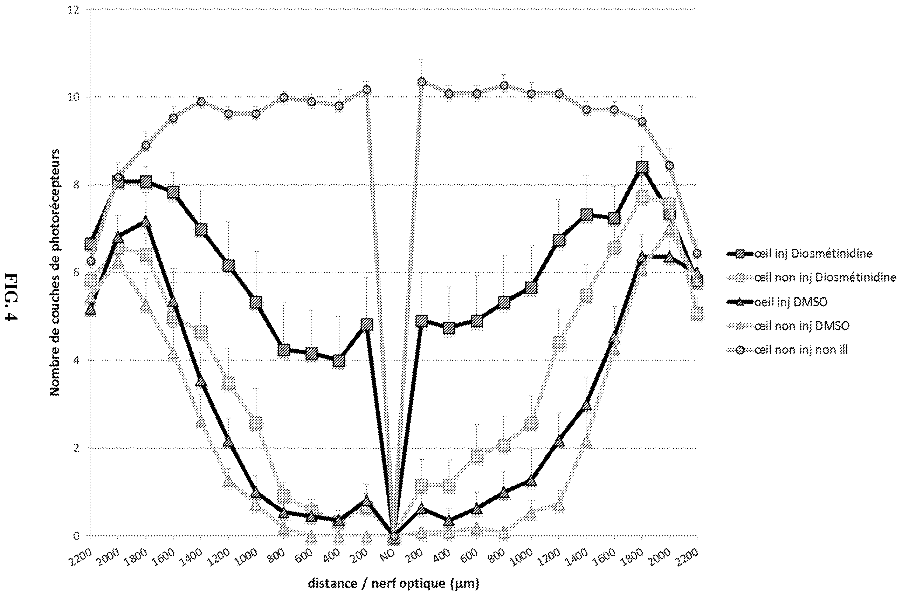

Les électrorétinogrammes réalisés 7 jours plus tard ont montré un effet protecteur de la diosmétinidine, dont la présence a permis de maintenir une activité électrique significative (Figures 2 et 3) et une bonne survie des photorécepteurs (Figure 4).

## Revendications

1. Composé de Formule I 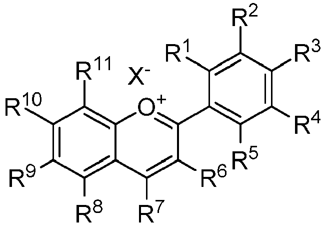 dans laquelle
**R¹, R², R³, R⁴** et **R⁵** représentent chacun indépendamment un groupement sélectionné parmi hydrogène, halo, hydroxyle, alkoxy, aryloxy, hétéroaryloxy, acyloxy, alkyle, aryle, aralkyle, alkylaryle, alcényle, nitro, nitrile, amino, avec la condition qu'au moins un de **R¹, R², R³, R⁴** ou **R⁵** représente un hydroxyle ;
**R⁶** représente un groupement sélectionné parmi hydrogène, halo, alkoxy, aryloxy, hétéroaryloxy, acyloxy, alkyle, aryle, aralkyle, alkylaryle, alcényle, nitro, nitrile, amino ;
**R⁷** représente un groupement sélectionné parmi hydrogène, halo, hydroxyle, alkoxy, aryloxy, hétéroaryloxy, acyloxy, alkyle, aryle, aralkyle, alkylaryle, alcényle, nitro, nitrile, amino ;
**R⁸, R⁹, R¹⁰** et **R¹¹** représentent chacun indépendamment un groupement sélectionné parmi hydrogène, halo, hydroxyle, alkoxy, aryloxy, hétéroaryloxy, acyloxy, alkyle, aryle, aralkyle, alkylaryle, alcényle, nitro, nitrile, amino, avec la condition qu'au moins un de **R⁸, R⁹, R¹⁰** ou **R¹¹** représente un hydroxyle ;
**X⁻** représente un anion sélectionné parmi : anion dérivé d'un acide minéral tel que par exemple un anion bromure, chlorure, borotétrafluorure ou perchlorure ; anion dérivé d'un acide organique, tel que par exemple un anion acétate, borate, citrate, tartrate, bisulfate, sulfate ou phosphate ; ou un anion dérivé d'un groupement sulfate ou sulfonate ;
pour son utilisation dans le traitement, la prévention et/ou la stabilisation de la DMLA, la maladie de Stargardt, la rétinopathie pigmentaire et/ou la rétinopathie diabétique.

2. Composé pour son utilisation selon la revendication **1,** dans lequel **R⁶** représente un atome d'hydrogène.

3. Composé pour son utilisation selon la revendication 1 ou la revendication 2, de Formule la 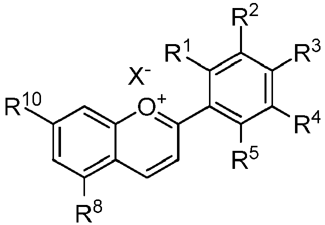 dans laquelle **R¹, R², R³, R⁴, R⁵, R⁸, R¹⁰** et **X⁻** sont tels que définis dans la revendication **1.**

4. Composé pour son utilisation selon l'une quelconque des revendications **1** à **3,** dans lequel :
**R¹, R², R³, R⁴** et **R⁵** représentent chacun indépendamment un groupement sélectionné parmi hydrogène, hydroxyle et alkoxy, avec la condition qu'au moins un de **R¹, R², R³, R⁴** ou **R⁵** représente un hydroxyle ;
**R⁸** et **R¹⁰** représentent chacun indépendamment un groupement sélectionné parmi hydrogène, hydroxyle et alkoxy, avec la condition qu'au moins un de **R⁸** ou **R¹⁰** représente un hydroxyle.

5. Composé pour son utilisation selon l'une quelconque des revendications **1** à **4,** de Formule Ib 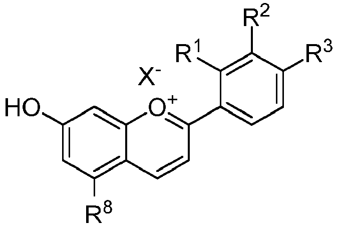 dans laquelle **R¹, R², R³, R⁸** et **X⁻** sont tels que définis dans la revendication **1.**

6. Composé pour son utilisation selon l'une quelconque des revendications **1** à **5,** dans lequel **R⁸** représente un atome d'hydrogène.

7. Composé pour son utilisation selon l'une quelconque des revendications **1** à **6,** sélectionné parmi :
chlorure de 2',7-dihydroxy-4'-méthoxy-flavylium ;
chlorure de 2',3',7-trihydroxy-4'-méthoxy-flavylium ;
chlorure de 3',7-dihydroxy-4'-méthoxy-flavylium ;
chlorure de 4',5,7-trihydroxy-flavylium ;
chlorure de 3',5,7-trihydroxy-4'-méthoxy-flavylium ;
chlorure de 3',4',5',5,7-pentadroxy-flavylium ;
chlorure de 3',4',5,7-tetrahydroxy-flavylium.

8. Composé sélectionné parmi :
chlorure de 2',7-dihydroxy-4'-méthoxy-flavylium ;
chlorure de 2',3',7-trihydroxy-4'-méthoxy-flavylium.

9. Composition pharmaceutique comprenant un composé selon la revendication **8** en combinaison avec un véhicule pharmaceutiquement acceptable.

10. Médicament comprenant un composé selon la revendication **8.**

## Patentansprüche

1. Verbindung der Formel I 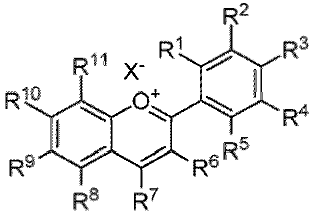 in welcher
**R¹, R², R³, R⁴** und **R⁵** jeweils unabhängig eine Gruppierung darstellen ausgewählt aus Wasserstoff, Halo, Hydroxyl, Alkoxy, Aryloxy, Heteroaryloxy, Acyloxy, Alkyl, Aryl, Aralkyl, Alkylaryl, Alkenyl, Nitro, Nitril, Amino, unter der Bedingung, dass wenigstens eines von **R¹, R², R³, R⁴** oder **R⁵** ein Hydroxyl darstellt;
**R⁶** eine Gruppierung darstellt ausgewählt aus Wasserstoff, Halo, Alkoxy, Aryloxy, Heteroaryloxy, Acyloxy, Alkyl, Aryl, Aralkyl, Alkylaryl, Alkenyl, Nitro, Nitril, Amino;
**R⁷** eine Gruppierung darstellt ausgewählt aus Wasserstoff, Halo, Hydroxyl, Alkoxy, Aryloxy, Heteroaryloxy, Acyloxy, Alkyl, Aryl, Aralkyl, Alkylaryl, Alkenyl, Nitro, Nitril, Amino;
**R⁸, R⁹, R¹⁰** und **R¹¹** jeweils unabhängig eine Gruppierung darstellen ausgewählt aus Wasserstoff, Halo, Hydroxyl, Alkoxy, Aryloxy, Heteroaryloxy, Acyloxy, Alkyl, Aryl, Aralkyl, Alkylaryl, Alkenyl, Nitro, Nitril, Amino, unter der Bedienung, dass wenigstens eines von **R⁸, R⁹, R¹⁰** oder **R¹¹** ein Hydroxyl darstellt;
**X⁻** ein Anion darstellt, ausgewählt aus: aus einer Mineralsäure gewonnenem Anion wie zum Beispiel einem Bromid-, Chlorid-, Borotetrafluorid- oder Perchloratanion; aus einer organischen Säure gewonnenem Anion, wie zum Beispiel einem Acetat-, Borat-, Citrat-, Tartrat-, Bisulfat-, Sulfat- oder Phosphatanion; oder einem Anion gewonnen aus einer Sulfat- oder Sulfonat-Gruppierung;
zur Verwendung in der Behandlung, der Vorbeugung und/oder der Stabilisierung der AMD, des Morbus Stargardt, der Pigment-Retinopathie und/oder der diabetischen Retinopathie.

2. Verbindung zur Verwendung nach Anspruch **1,** in welcher **R⁶** ein Wasserstoffatom darstellt.

3. Verbindung zur Verwendung nach Anspruch **1** oder Anspruch **2** der Formel Ia 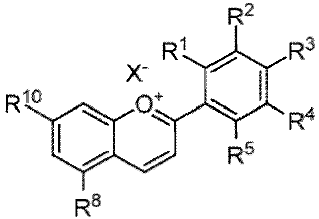 in welcher **R¹, R², R³, R⁴, R⁵, R⁸, R¹⁰** und **X⁻** solcherart sind, wie in Anspruch **1** definiert.

4. Verbindung zur Verwendung nach einem der Ansprüche **1** bis **3,** in welcher:
**R¹, R², R³, R⁴** und **R⁵** jeweils unabhängig eine Gruppierung darstellen ausgewählt aus Wasserstoff, Hydroxyl und Alkoxy, unter der Bedingung, dass wenigstens eines von **R¹, R², R³, R⁴** oder **R⁵** ein Hydroxyl darstellt;
**R⁸** und **R¹⁰** jeweils unabhängig eine Gruppierung darstellen ausgewählt aus Wasserstoff, Hydroxyl und Alkoxy, unter der Bedingung, dass wenigstens eines von **R⁸** oder **R¹⁰** ein Hydroxyl darstellt.

5. Verbindung zur Verwendung nach einem der Ansprüche **1** bis **4** der Formel **Ib** 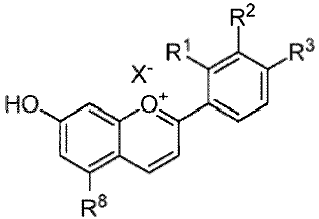 in welcher **R¹, R², R³, R⁸** und **X⁻** solcherart sind, wie in Anspruch **1** definiert.

6. Verbindung zur Verwendung nach einem der Ansprüche **1** bis **5,** in welcher **R⁸** ein Wasserstoffatom darstellt.

7. Verbindung zur Verwendung nach einem der Ansprüche **1** bis **6,** ausgewählt aus:
Chlorid von 2',7-Dihydroxy-4'-Methoxy-Flavylium;
Chlorid von 2',3',7-Trihydroxy-4'-Methoxy-Flavylium;
Chlorid von 3',7-Dihydroxy-4'-Methoxy-Flavylium;
Chlorid von 4',5,7-Trihydroxy-Flavylium;
Chlorid von 3',5,7-Trihydroxy-4'-Methoxy-Flavylium;
Chlorid von 3',4',5',5,7-Pentadroxy-Flavylium;
Chlorid von 3',4',5,7-Tetrahydroxy-Flavylium.

8. Verbindung, ausgewählt aus:
Chlorid von 2',7-Dihydroxy-4'-Methoxy-Flavylium;
Chlorid von 2',3',7-Trihydroxy-4'-Methoxy-Flavylium.

9. Pharmazeutische Zusammensetzung, umfassend eine Verbindung nach Anspruch **8** in Kombination mit einem geeigneten pharmazeutischen Trägerstoff.

10. Medikament, umfassend eine Verbindung nach Anspruch **8.**

## Claims

1. Compound of Formula I 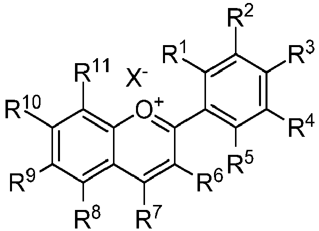 wherein
**R¹, R², R³, R⁴** and **R⁵** each independently represent a group selected from hydrogen, halo, hydroxyl, alkoxy, aryloxy, heteroaryloxy, acyloxy, alkyl, aryl, aralkyl, alkylaryl, alkenyl, nitro, nitrile, amino, with the condition that at least one of **R¹, R², R³, R⁴** or **R⁵** represents hydroxyl;
**R⁶** represents a group selected from hydrogen, halo, alkoxy, aryloxy, heteroaryloxy, acyloxy, alkyl, aryl, aralkyl, alkylaryl, alkenyl, nitro, nitrile, amino;
**R⁷** represents a group selected from hydrogen, halo, hydroxyl, alkoxy, aryloxy, heteroaryloxy, acyloxy, alkyl, aryl, aralkyl, alkylaryl, alkenyl, nitro, nitrile, amino;
**R⁸, R⁹, R¹⁰** and **R¹¹** each independently represent a group selected from hydrogen, halo, hydroxyl, alkoxy, aryloxy, heteroaryloxy, acyloxy, alkyl, aryl, aralkyl, alkylaryl, alkenyl, nitro, nitrile, amino, with the condition that at least one of **R⁸, R⁹, R¹⁰** or **R¹¹** represents hydroxyl;
**X⁻** represents an anion selected from: anion derived from a mineral acid such as for example a bromide, chloride, borotetrafluoride or perchloride anion; anion derived from an organic acid such as for example an acetate, borate, citrate, tartrate, bisulphate, sulphate or phosphate anion; or anion derived from a sulphate or sulphonate group;
for use in the treatment, prevention and/or stabilization of AMD, Stargardt's disease, pigmentary retinopathy and/or diabetic retinopathy.

2. Compound for use according to claim **1,** wherein **R⁶** represents a hydrogen atom.

3. Compound for use according to claim **1** or claim **2,** of Formula Ia 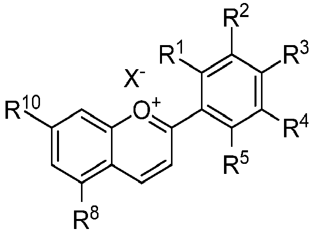 wherein **R¹, R², R³, R⁴, R⁵, R⁸, R¹⁰** and **X⁻** are as defined in claim **1.**

4. Compound for use according to any one of claims **1** to **3,** wherein:
**R¹, R², R³, R⁴** and **R⁵** each independently represent a group selected from hydrogen, hydroxyl and alkoxy, with the condition that at least one of **R¹, R², R³, R⁴** or **R⁵** represents hydroxyl;
**R⁸** and **R¹⁰** each independently represent a group selected from hydrogen, hydroxyl and alkoxy, with the condition that at least one of **R⁸** or **R¹⁰** represents hydroxyl.

5. Compound for use according to any one of claims **1** to **4,** of Formula Ib 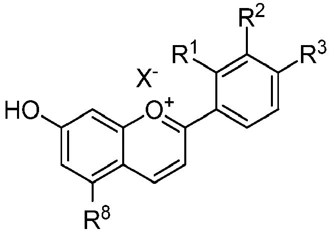 wherein **R¹, R², R³, R⁸** and **X⁻** are as defined in claim **1.**

6. Compound for use according to any one of claims **1** to **5,** wherein **R⁸** represents a hydrogen atom.

7. Compound for use according to any one of claims **1** to **6,** selected from:
2',7-dihydroxy-4'-methoxy-flavylium chloride;
2',3',7-trihydroxy-4'-methoxy-flavylium chloride;
3',7-dihydroxy-4'-methoxy-flavylium chloride;
4',5,7-trihydroxy-flavylium chloride;
3',5,7-trihydroxy-4'-methoxy-flavylium chloride;
3',4',5',5,7-pentadroxy-flavylium chloride;
3',4',5,7-tetrahydroxy-flavylium chloride.

8. Compound selected from:
2',7-dihydroxy-4'-methoxy-flavylium chloride;
2',3',7-trihydroxy-4'-methoxy-flavylium chloride.

9. Pharmaceutical composition comprising a compound according to claim **8** in combination with a pharmaceutically acceptable carrier.

10. Medicament comprising a compound according to claim **8.**
